Europäisches Patentamt

European Patent Office

Office européen des brevets

(19)

(11) Veröffentlichungsnummer: **0 194 344**
**B1**

(12) **EUROPÄISCHE PATENTSCHRIFT**

(45) Veröffentlichungstag der Patentschrift:
14.12.88

(21) Anmeldenummer: **85114131.7**

(22) Anmeldetag: **06.11.85**

(51) Int. Cl.⁴: **G 01 N 27/56**

(54) Verfahren und Vorrichtung zur Konditionierung eines Gassensors.

(30) Priorität: **26.02.85 DE 3506688**

(43) Veröffentlichungstag der Anmeldung:
**17.09.86 Patentblatt 86/38**

(45) Bekanntmachung des Hinweises auf die Patenterteilung:
**14.12.88 Patentblatt 88/50**

(84) Benannte Vertragsstaaten:
**CH DE GB LI NL**

(56) Entgegenhaltungen:
**DE-A-2 203 942**
**US-A-3 689 222**
**US-A-4 376 681**
**US-A-4 469 562**

(73) Patentinhaber: **Drägerwerk Aktiengesellschaft,
Moislinger Allee 53- 55, D-2400 Lübeck 1 (DE)**

(72) Erfinder: **Lungu, Mihail, Dr. Dipl.- Chem.,
Eichenweg 17, D-2067 Reinfeld (DE)**
Erfinder: **Rogge, Bernd, Dr. Dipl.- Chem., Tiefe
Mark 66, D-4600 Dortmund 41 (DE)**

LIBER, STOCKHOLM 1988

## Beschreibung

Die Erfindung betrifft ein Verfahren zur Konditionierung eines Gassensors, insbesondere zur Messung des $CO_2$-Partialdruckes, dessen Elektrodenanordnung von einem Elektrolyten umgeben und gegenüber der Atmosphäre mit einer gasdurchlässigen Membran abgeteilt ist, und über diese Membran einer ein Kalibriergas enthaltenden Umgebung ausgesetzt wird, wobei das Kalibriergas in isotonem Gleichgewicht mit dem Elektrolyten gehalten wird. Außerdem wird ein Gassensor mit einer zur Durchführung des Verfahrens geeigneten Auflage angegeben.

Ein derartiges Verfahren wird in der DE-OS-2 203 942 beschrieben.

Dort wird ein Sensor zur Messung des $CO_2$-Partialdruckes angegeben, dessen meßempfindlicher Teil aus einer Elektrodenanordnung besteht, die von einem Elektrolyten umgeben ist, welcher von einer $CO_2$- und wasserdampfdurchlässigen Membran von der Umgebung abgeteilt ist. Die Elektrodenanordnung mißt den pH-Wert des Elektrolyten, der sich infolge Diffusion von $CO_2$ aus der Umgebungsatmosphäre durch die Membran in den Elektrolyten ändert. Wenn der Sensor der Umgebungsatmosphäre ungeschützt ausgesetzt ist, wie dies beispielsweise während der Lagerung oder auch während der Meßpausen zwischen zwei Messungen der Fall ist, entweicht aus dem Elektrolyten Wasserdampf durch die Membran in die Umgebung. Wird ein solcher Sensor zur Messung des $CO_2$-Partialdruckes auf erhöhter Betriebstemperatur gehalten, wie es zum Beispiel bei der transcutanen Messung des $CO_2$-Partialdruckes des Blutes notwendig ist, diffundiert aus einem Elektrolyten, welcher gelöste Karbonate enthält, zusätzlich gasförmiges $CO_2$ in die Umgebungsatmosphäre. Durch den Verlust von Wasserdampf und $CO_2$ aus dem Elektrolyten verschiebt sich der Arbeitspunkt des $CO_2$-Sensors, da durch die Diffusion von $CO_2$ aus dem Elektrolyten dieser alkalisch wird und somit eine Änderung des pH-Wertes auftritt. Der Sensor wird dann außerhalb des spezifizierten Meßbereichs betrieben, wo durch die Anzeige verfälscht und das Meßergebnis unbrauchbar werden kann. Alkalische Elektrolyte sind für Glaselektroden, wie sie beispielsweise in der transcutanen Meßtechnik benutzt werden, schädlich und verändern die Sensoreigenschaften bezüglich Empfindlichkeit und Ansprechzeit nachteilig. Bevor eine $CO_2$-Messung mit dem bekannten Sensor durchgeführt werden kann, wird dieser mit einer bekannten $CO_2$-Konzentration kalibriert. Dazu wird der Sensor einer Gasmischung aus Kohlendioxid und Luft von festgelegter Zusammensetzung ausgesetzt. Die Partialdrücke von Wasserdampf und $CO_2$ des Kalibriergases sollen mit den entsprechenden Partialdrücken im Elketrolyten übereinstimmen, so daß eine Diffusion von Wasserdampf und $CO_2$ aus dem Elektrolyten verhindert wird.

Eine solche Kalibrierung ist jedoch aufwendig und kostspielig, da ständig eine separate Kalibriereinheit bereitgehalten werden muß, welche zur Aufrechterhaltung der einmal eingestellten Gaskonzentrationen mit dem Kalibriergas gespült werden muß. Außerdem wird die Handhabung des $CO_2$-Sensors dadurch erschwert, daß vor der Durchführung einer Messung der $CO_2$-Sensor aus dem Kalibrierbehälter genommen werden muß, bevor er an dem Meßort angebracht werden kann.

Die Erfindung geht von der Aufgabe aus, ein Verfahren der genannten Art derart zu verbessern, daß unter Beibehaltung der Betriebsbedingungen des Sensors und ohne diesen in eine das Kalibriergas enthaltende Umgebung einbringen zu müssen, die Zusammensetzung des Elektrolyten nicht wesentlich verändert wird. Außerdem soll ein Gassensor angegeben werden, mit dem ein verbessertes Konditionierungsverfahren durchgeführt werden kann.

Die Lösung der Aufgabe erfolgt dadurch, daß die Membran des Sensors mit einer gas- und wasserdampfdichten Auflage unter Bildung eines Zwischenraumes abgedeckt und daß das isotone Gleichgewicht durch das aus dem Elektrolyten durch die Membran bis zu der Auflage diffundierende Gas erzeugt und aufrechterhalten wird.

Auf diese Weise ist es möglich, den Gassensor während der Meßpausen oder einer länger andauernden Lagerung unter Aufrechterhaltung der Betriebsbedingungen in einer Bereitschaftstellung zu halten, während der trotz einer erhöhten Betriebstemperatur die Zusammensetzung des Elektrolyten nur unbedeutend verändert wird, so daß eine Gasmessung innerhalb eines vorgeschriebenen Kalibrierintervalls ohne eine zusätzliche Kalibrierung erfolgen kann bzw. ein Abwarten einer Einlaufzeit nach Inbetriebnahme des einmal ausgeschalteten Sensors nicht erforderlich ist. Dazu ist lediglich die Auflage von der Membran des Sensors zu entfernen. Der Sensor ist dann sofort meßbereit.

Durch die Anwendung des erfindungsgemäßen Verfahrens wird der Elektrolyt des Sensors vorteilhaft gegenüber seiner Umgebung isoliert, so daß der extrem geringvolumige Raum zwischen Auflage und Meßelektrode sich z. B. mit $CO_2$ und Wasserdampf sättigen kann, wodurch sich der Elektrolyt in einer isotonen Gleichgewichts-umgebung befindet. Das im Elektrolyten gelöste $CO_2$ reicht aus, um dieses Volumen mit $CO_2$ zu sättigen, so daß der Raum zwischen Auflage und Meßelektrode gewissermaßen als Kalibriervolumen angesehen werden kann. Dabei sind die für den Betrieb eines Sensors zur transcutanen Bestimmung von $CO_2$ notwendigen Temperaturen von 37 bis 44°C für die Aufrechterhaltung dieses Gleichgewichtes nicht schädlich.

Ein Gassensor, mit dem das Verfahren durchgeführt werden kann, enthält eine Elektrodenanordnung in einem Elektrolyten, der über eine Membran der Umgebung ausgesetzt ist. Zum Zwecke der Konditionierung wird diese Membran mit einer gas- und wasserdampfdichten Auflage unter Bildung eines Zwischenraumes abgedeckt. Bei abgedeckter Membran kann sich in dem Zwischenraum das isotone Gleichgewicht zwischen $CO_2$ und Wasserdampf im Elektrolyten einerseits und im Gasraum des Zwischenraumes andererseits ausbilden.

Als Auflage kann eine kaschierte Aluminiumfolie mit einer Klebeschicht versehen sein, welche eine die Membran ausnehmende Öffnung aufweist. Der Sensor wird nach einer durchgeführten Messung abgedeckt, indem die Auflage mittels der Klebeschicht auf die Sensorumrandung geklebt wird, wodurch die Membran des Sensors wasserdampf- und gasdicht abgedeckt wird. Die Aluminiumfolie verhindert eine Diffusion von Wasserdampf und Gas aus dem Elektrolyten in die Umgebungsatmosphäre auch bei erhöhten Betriebstemperaturen von beispielsweise 37 bis 44° C. Während der gesamten Bereitstellungszeit verbleibt die Aluminiumfolie auf dem Sensor, wobei keine Einschränkungen bezüglich der Dauer der Bereitstellungszeit gegeben sind.

Als eine günstige Kaschierung für die Aluminiumfolie hat sich Polyethylen erwiesen.

Darüberhinaus kann zur besseren Handhabung die Klebeschicht in einem bestimmten Bereich mit einer nichtklebenden Beschichtung versehen sein, wodurch es dem Benutzer ermöglicht wird, die Auflage auf einfache Weise von dem Sensor wieder zu entfernen, sobald die Bereitschaftszeit beendet ist.

Die nichtklebende Beschichtung kann vorzugsweise aus Silikon oder aus silikonisiertem Papier sein.

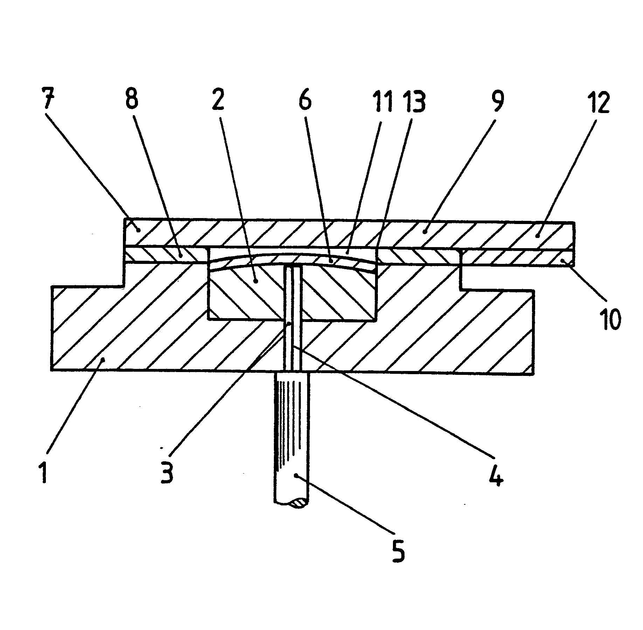

Ein Gassensor mit einer Auflage zur Durchführung des Verfahrens wird anhand der Zeichnung schematisch dargestellt und im folgenden erläutert.

In der einzigen Figur ist ein $CO_2$-Sensor dargestellt, in dessen Meßkopf 1 die Elektrodenanordnung 2 angeordnet ist, welche eine Glaselektrode 3 aufweist, in der ein Draht 4 zu einer Meßleitung 5 geführt ist. Die Elektrodenanordnung 2 ist durch eine $CO_2$ und wasserdampfdurchlässige Membran 6 abgeschlossen, wobei sich zwischen Membran 6 und Elektrodenanordnung 2 der nichtdargestellte Elektrolyt befindet. Der Meßkopf 1 ist membranseitig von der Auflage 7 abgeschlossen und mit der ringförmigen, eine Öffnung 13 besitzenden Klebeschicht 8 an dem Meßkopf 1 befestigt. Den $CO_2$- und wasserdampfundurchlässigen Teil der Auflage 7 bildet die Aluminiumfolie 9, an deren überstehenden Bereich 12 eine nichtklebende Beschichtung 10 angebracht ist.

Nachdem die Auflage 7 auf den Meßkopf 1 aufgebracht wurde, wird der zwischen Aluminiumfolie 9 und Membran 6 gebildete Zwischenraum 11 von Wasserdampf und $CO_2$ aus dem Elektrolyten gesättigt, so daß ein Gleichgewicht der Partialdrücke von Wasserdampf und $CO_2$ zwischen dem Elektrolyten und dem Zwischenraum 11 herrscht, und dadurch die Zusammensetzung des Elektrolyten während der Bereitschaftszeit nicht mehr verändert wird.

**Patentansprüche**

1. Verfahren zur Konditionierung eines Gassensors, insbesondere zur Messung des $CO_2$-Partialdruckes, dessen Elektrodenanordnung (2) von einem Elektrolyten umgeben und gegenüber der Atmosphäre mit einer gasdurchlässigen Membran abgeteilt ist und über diese Membran (6) einer ein Kalibriergas enthaltenden Umgebung ausgesetzt wird, wobei das Kalibriergas in isotonem Gleichgewicht mit dem Elektrolyten gehalten wird, dadurch gekennzeichnet, daß die Membran (6) des Sensors mit einer gas- und wasserdampfdichten Auflage (7) unter Bildung eines Zwischenraumes (11) abgedeckt und daß das isotone Gleichgewicht durch das aus dem Elektrolyten durch die Membran (6) bis zu der Auflage (7) diffundierende Gas erzeugt und aufrechterhalten wird.

2. Gassensor zur Messung von Komponenten eines Gasgemisches, insbesondere zur Messung von $CO_2$ in Luft, mit einer Elektrodenanordnung (2) in einem Elektrolyten, der über eine Membran (6) zum Zwecke der Konditionierung einer ein Kalibriergas enthaltenden abgeschlossenen Umgebung ausgesetzt ist, dadurch gekennzeichnet, daß die Membran (6) mit einer gas- und wasserdampfdichten Auflage (7) unter Bildung eines Zwischenraumes (11) abgedeckt ist.

3. Gassensor nach Anspruch 2, dadurch gekennzeichnet, daß als Auflage (7) eine kaschierte Aluminiumfolie (9) mit einer Klebeschicht (8) versehen ist, welche eine die Membran (6) ausnehmende Öffnung (13) aufweist.

4. Auflage nach Anspruch 3, dadurch gekennzeichnet, daß die Aluminiumfolie (9) mit Polyethylen kaschiert ist.

5. Auflage nach Anspruch 3, dadurch gekennzeichnet, daß die Klebeschicht (8) einen Bereich (12) besitzt, welcher mit einer nichtklebenden Beschichtung (10) versehen ist.

6. Auflage nach Anspruch 5, dadurch gekennzeichnet, daß die nichtklebende Beschichtung (10) Silikon enthält.

**Claims**

1. Process for the conditioning of a gas sensor, particularly for measuring the partial pressure of

$CO_2$, whose electrode arrangement (2) is surrounded by an electrolyte and is separated from the atmosphere by a gas-permeable membrane and is exposed via this membrane (6) to surroundings containing a calibrating gas, the calibrating gas being held in isotonic equilibrium with the electrolyte,

characterised in that the membrane (6) of the sensor is covered with a coating (7) sealed against gas and water, whereby a gap (11) is formed, and in that the isotonic equilibrium is produced and maintained by the gas which diffuses from the electrolyte through the membrane (6) to the coating (7).

2. Gas sensor for measuring components of a gas mixture, particularly for measuring $CO_2$ in air, this sensor having an electrode arrangement (2) in an electrolyte and being exposed to surroundings containing a calibrating gas via a membrane (6) for the purpose of conditioning,

characterised in that the membrane (6) is covered with a coating sealed against gas and water, whereby a gap (11) is formed.

3. Gas sensor according to claim 2, characterised in that a coated aluminium foil (9) having an adhesive layer (8) is provided as a coating (7), this adhesive layer having an aperture which excludes the membrane (6).

4. Coating according to claim (3), characterised in that the aluminium foil (9) is coated with polyethylene.

5. Coating according to claim 3, characterised in that the adhesive layer (8) has a region (12) which is provided with a non-adhesive coating (10).

6. Coating according to claim 5, characterised in that the non-adhesive coating (10) contains silicon.

**Revendications**

1. Procédé de conditionnement d'un détecteur de gaz spécialement destiné à la mesure de la pression partielle en $CO_2$, dont la structure d'électrodes (2) est entourée d'un électrolyte et séparée de l'atmosphère par une membrane perméable aux gaz et est exposée, par l'intermédiaire de cette membrane (6), à une atmosphère renfermant un gaz d'étalonnage, le gaz d'étalonnage étant maintenu en équilibre isotonique avec l'électrolyte, caractérisé en ce que la membrane (6) du détecteur est recouverte d'une couche imperméable aux gaz et à l'eau, avec formation d'une chambre intermédiaire (11) et en ce que l'équilibre isotonique est obtenu et maintenu grâce au gaz diffusant depuis l'électrolyte par la membrane (6) jusqu'à la couche (7).

2. Détecteur de gaz pour la mesure des composants d'un mélange gazeux, spécialement pour la mesure du $CO_2$ dans l'air, avec une structure d'électrodes (2) dans un électrolyte qui est exposé, par l'intermédiaire d'une membrane (6), dans un but de conditionnement, à une atmosphère fermée renfermant un gaz d'étalonnage, caractérisé en ce que la membrane (6) est recouverte d'une couche (7) imperméable aux gaz et à l'eau, avec formation d'une chambre intermédiaire (11).

3. Détecteur de gaz selon la revendication 2, caractérisé en ce que la couche (7) est un film d'aluminium (9) contrecollé, pourvu d'une couche de colle (8) qui présente une ouverture (13) dégageant la membrane (6).

4. Couche selon la revendication 3, caractérisée en ce que le film d'aluminium (9) est contrecollé à du polyéthylène.

5. Couche selon la revendication 3, caractérisée en ce que la couche de colle (8) comporte une zone (12) revêtue d'un revêtement (10) non collant.

6. Couche selon la revendication 5, caractérisée en ce que le revêtement non collant renferme du silicone.